Europäisches Patentamt

**European Patent Office**

**Office européen des brevets**

(11) Publication number: **0 097 060**
A2

# EUROPEAN PATENT APPLICATION

(21) Application number: **83303471.3**

(22) Date of filing: **15.06.83**

(51) Int. Cl.³: **A 61 M 16/00**

(30) Priority: **15.06.82 GB 8217354**

(43) Date of publication of application: **28.12.83**
**Bulletin 83/52**

(84) Designated Contracting States: **DE FR GB IT NL SE**

(71) Applicant: **ELECTRONIC PNEUMATIC APPARATUS & CONTROLS LIMITED, The Lanterns 24 Worrin Road, Shenfield Brentwood Essex (GB)**

(72) Inventor: **Burchell, Geoffrey Barnett, Dr., The Lanterns 24 Worrin Road, Shenfield Brentwood Essex (GB)**

(74) Representative: **Burrows, Anthony Gregory et al, Haseltine Lake & Co. Hazlitt House 28 Southampton Buildings Chancery Lane, London, WC2 1AT (GB)**

(54) **Improvements in or relating to respiratory apparatus.**

(57) A resuscitation apparatus comprises a respirable gas flow valve (REDUCING VALVE), one or more modular control devices (PB, UO, AC) connected to the valve (REDUCING VALVE) for operating the valve, and a modular administering device (IA, IUO, PVA, BV) connected to downstream of the valve (REDUCING VALVE) for administering the gas to a patient's respiratory system. The control device(s) can be selected from two manual control devices (PB, UO), a series of pneumatic automatic control devices (AC-PNEUMATIC), and a series of electronic automatic control devices (AC-ELECTRONIC); whilst the administering device can be selected from a series of injection airways (IA), a series of oxygen injection units (IUO), and a series of patient valves (PVA, BV).

-1-

IMPROVEMENTS IN OR RELATING TO

RESPIRATORY APPARATUS

This invention relates to respiratory apparatus, in particular resuscitation apparatus.

Resuscitation apparatus is used to resuscitate a person whose respiration has become insufficient to maintain life for any substantial period of time. The resuscitation apparatus used depends on the particular patient (in particular his or her size) and the circumstances in which the patient is found. A large number of different types of resuscitation apparatus have thus been developed to meet the various different situations that arise in practice. However, known resuscitation apparatus tend to be of limited utility.

It is an aim of the present invention to provide versatile resuscitation apparatus.

According to a first aspect of the present invention, there is provided resuscitation apparatus comprising an inlet connectible to a source of respirable gas, an outlet connectible to a patient's respiratory system, duct means interconnecting said inlet and said outlet, a valve interposed in said duct means and operable selectively manually and automatically, manually operable means connected to said valve and operable manually to operate said valve, and automatically operable means connected to said valve and operable automatically to operate said valve.

According to a second aspect of the present invention there is provided resuscitation apparatus comprising a housing; an inlet port in a wall of the housing connectible to a source of respirable gas; an outlet port in a wall of the housing connectible to means for administering respirable gas to a patient; a flow pasasge extending within the housing from the inlet port to the outlet port; a valve in said passage, the valve being capable of being operated manually or automatically; means operable manually from outside of the housing for opening and closing the valve; and a connector in the wall of the housing associated with the valve and suitable for connecting the valve to external means for operating the valve automatically.

The resuscitation apparatus is thus capable of being operated manually or automatically to supply pulses of respirable gas to a patient.

The connector may be electrical and the valve have a solenoid actuator so that electronic control means may be employed to operate the valve. There is provided an electronic control device for operating the valve automatically, this device having an electrical connector engageable with the electrical connector of the apparatus.

The control device may have its own power supply or may be connectible to a mains electricity supply. The control device preferably has a housing containing suitable electronic control circuits and a power supply if it is not to be operated from a mains electricity supply. The control circuits are adapted to generate electrical signals which effect opening and closing of the valve, preferably at a variable frequency. Each period for which the valve is open corresponds to the inspiratory phase of a patient's breathing, and each period for which the valve is closed corresponds to the expiratory phase of a patient's breathing. The control device is preferably adapted to give a constant inspiratory phase/expiratory phase ratio, i.e. the ratio of the duration of each pulse of gas to the interval between consecutive pulses is a constant. Preferably, the ratio is 1:2.

It is also preferred that the duration of each pulse of gas can be varied by manual setting of a suitable control. This makes it possible for the resuscitation apparatus to supply pulses of respirable gas that can be adjusted to suit the size (or lung capacity) of the patient be he or she a newly born baby or a fully grown adult with a relatively large lung capacity. Typically, the range of settings of the manual control may vary the frequency of the gas pulses from 10 or 15 to 40 per minute.

It is to be appreciated from the aforegoing description that the resuscitation apparatus may be set to operate automatically to produce gas pulses at a chosen frequency by using the control device. It is, however, a feature of the resuscitation apparatus that it can be operated entirely manually and without connecting a control device thereto. This can be done by operating the said manually operable means for opening and closing the valve (which means is conveniently a push-button). Manual operation of the valve offers flexibility in that it is possible to produce any desired pattern of gas pulses. The resuscitation apparatus is thus usable even if there is no available power supply or automatic means for operating the valve or if the condition of the patient is so serious that there is no time to connect the resuscitation apparatus to any available means for operating the valve automatically. Moreover, the valve is preferably of a kind such that when operating automatically, the control signals it recieves are able to be manually over-ridden.

It is another feature of the resuscitation apparatus that its outlet port may be connected to any suitable means for delivering respirable gas to a patient. Such means may comprise an artificial airway. A preferred artificial airway is as claimed and described in UK Patent Specification No. 1 558 171. Such an artificial airway is anatomically-shaped and has a flange at one end to limit its insertion into a patient's airway and a fixed injector at the flanged end for injecting respirable gas at relatively high flow rates into the airway to entrain air from the atmosphere. The length of the airway is related to the size of the patient so that the inner end of the airway, when the airway is in position, lies in the oral pharynx. Such an artificial airway obviates the need for a face mask and thus overcomes difficulty in keeping an air-tight fit between the face mask and the patient's face. The size of the artificial airway selected will depend on the size of the patient. It is alternatively possible to use more conventional means (including face masks) to deliver the respirable gas to the patient. Thus, the outlet port may be connected to a conventional non-return valve which is fitted with a suitable taper (or face mask). The non-return valve may be suitable for a child and fitted with a British Standard paediatric taper or maybe suitable for an adult and fitted with the British Standard adult taper. In both instances, there may be a flow control valve in the conduit connecting the outlet port to the non-return valve. Other alternative means for delivering respirable gas to the patient that are connectible to the outlet port include endotracheal and trachaeostomy tubes.

The resuscitation apparatus preferably has in a wall of the housing a second outlet port communicating with the flow passage in the housing upstream of the valve. The second outlet port may be used to supply respirable gas continuously to a second patient while resuscitating a first. If desired, the second outlet port may be connected to a manually operable valve, downstream thereof, to produce a second pulsed supply of respirable gas. By connecting the outlet of such manually operable valve to a means for delivering respirable gas to a patient such as has been described above it is possible to use the resuscitation apparatus to resuscitate two patients (e.g. mother and child) simultaneously, generally with the valve within the housing being operated automatically. It is alternatively possible to attach the outlet of the manually-operable valve connected to the second outlet port to an inflatable pillow, which may then be inflated to a desired degree and used to prop the head of a patient so as to facilitate delivery of the respirable gas.

According to a third aspect of the present invention, there is provided a method of assembling a resuscitation apparatus, comprising selecting a control device from a range of modular control devices, connecting said control device to a respirable gas flow control valve to be operated by said control device, selecting a respirable gas administering device from a range of modular respirable gas administering devices, and connecting said administering device to downstream of said valve for administering said gas to a patient's respiration system.

According to a fourth aspect of the present invention, there is provided a collection of modular devices from a selection from which a resuscitation apparatus can be assembled, comprising a respirable gas flow valve, a range of modular control devices each connectible to said valve for opening the same, and a range of modular respirable gas administering devices connectible to downstream of said valve for administering said gas to a patient's respiratory system.

According to a fifth aspect of the present invention, there is provided a resuscitation apparatus, comprising a respirable gas flow valve, a modular control device connected to said valve for operating the same, and a modular respirable gas administering device connected to downstream of said valve for administering said gas to a patient's respiratory system.

According to a sixth aspect of the present invention, there is provided resuscitation apparatus comprising an inlet connectible to a source of respirable gas, an outlet connectible to a patient's respiratory system, duct means interconnecting said inlet and said outlet, a valve interposed in said duct means and operable automatically, and electrical means connected to said valve and arranged to operate said valve automatically, said electrical means including electrical switching means arranged to detect the presence or absence of said gas under pressure upstream of said valve and accordingly to energize or de-energize said electrical means.

According to a seventh aspect of the present invention, there is provided a respiratory apparatus including first and second variable flow control valves which serve to control respective flows of gaseous fluid therethrough, and a non-pneumatic adjusting linkage interconnecting the valves so that adjustment of the first valve is accompanied by adjustment of the second valve.

According to an eighth aspect of the present invention, there is provided a valve device, comprising an inlet for fluid, an outlet for said fluid, valve closure means arranged to control flow of said fluid from said inlet to said outlet, and a pilot pressure chamber bounded by said valve closure means, the effective area of said valve closure means exposed to the pressure of said fluid between said inlet and said outlet being less than the effective area of said valve closure means exposed to the pressure in said chamber.

According to a ninth aspect of the present invention, there is provided a valve device comprising a valve housing, a valve closure member movable in said housing from an open position to a closed position, an inlet to said housing for fluid, and an outlet from said housing for fluid, the arrangement being such that, as said member moves from said open position to said closed position, initially fluid can flow via a first path from said inlet to a limited degree and via a second path from said inlet to no more than a limited extent, but subsequently said member enables flow of said fluid _via_ said second path from said inlet to greater than said limited extent, whereby said fluid can flow from said inlet to an increased extent.

According to a tenth aspect of the present invention, there is provided a valve device comprising a valve housing, a valve closure member movable in said housing from an open position to a closed position, a tubular inlet to said housing for fluid, and an outlet from said housing for fluid, said member being in the form of a bobbin closely encircling said tubular inlet and including first and second flanges of substantially equal external dimension transverse to the bobbin axis.

According to an eleventh aspect of the present invention, there is provided a valve device comprising a first valve including a first valve closure member arranged to open automatically upon occurrence of a predetermined pressure differential thereacross, a second valve which includes a selectively closable second valve closure member and through which the first valve exhausts when the second valve closure member is open, and a third valve which includes a third valve closure member arranged to open automatically upon occurrence of a predetermined pressure differential thereacross and through which the first valve exhausts when the second valve closure member is closed, the arrangement being such that pressure upstream of said first valve at which said third valve opens is higher than that pressure upstream of said first valve at which said first valve opens.

Resuscitation apparatus according to the present invention will now be described by way of example with reference to the accompanying drawings, in which:-

Figure 1 is a schematic perspective view of one embodiment of resuscitation apparatus according to the invention;

Figure 2 is a schematic perspective view of one embodiment of a control device adapted to be connected to the resuscitation apparatus shown in Figure 1;

Figure 3 is a schematic side elevation of the apparatus shown in Figure 1 illustrating the inlet port;

Figure 4 is a schematic view from below of the apparatus shown in Figure 1 illustrating the electrical connector;

Figure 5 is a schematic circuit diagram of the apparatus shown in Figure 1;

Figure 6 is a graph showing the gas supply that the apparatus shown in Figure 1 is capable of supplying;

Figure 7 is a side view of an anatomically-shaped artificial airway suitable for use with the resuscitation apparatus shown in Figure 1;

Figure 8 is an end view of the airway shown in Figure 7;

Figures 9 to 13 all illustrate devices connectible to one or other of the outlet ports of the apparatus shown in Figure 1;

Figure 14 is a diagrammatic representation of a manner in which the resuscitation apparatus can be assembled by a method involving selection from ranges of modular devices;

Figure 15 shows a diagram of an electronic automatic control device for giving variable respiration frequency and a fixed inhalation/exhalation time ratio;

Figure 16 is a diagram of part of a modified version of that device;

Figure 17 is a diagram of part of another modified version of that device;

Figure 18 is a diagram of part of a further modified version of that device;

Figure 19 is a diagram of a pneumatic part of a further embodiment of the apparatus;

Figure 20 is a diagram of a mechanical part of that further embodiment;

Figure 21 shows an axial section through a patient valve of that further embodiment;

Figure 22 shows an axial section through part of a modified version of that patient valve;

Figure 23 shows an axial section through a normally-open, pilot-controlled valve seen in Figure 19, and

Figure 24 shows an axial section through a normally-closed, pilot-controlled valve seen in figure 19.

The drawings are not to scale.

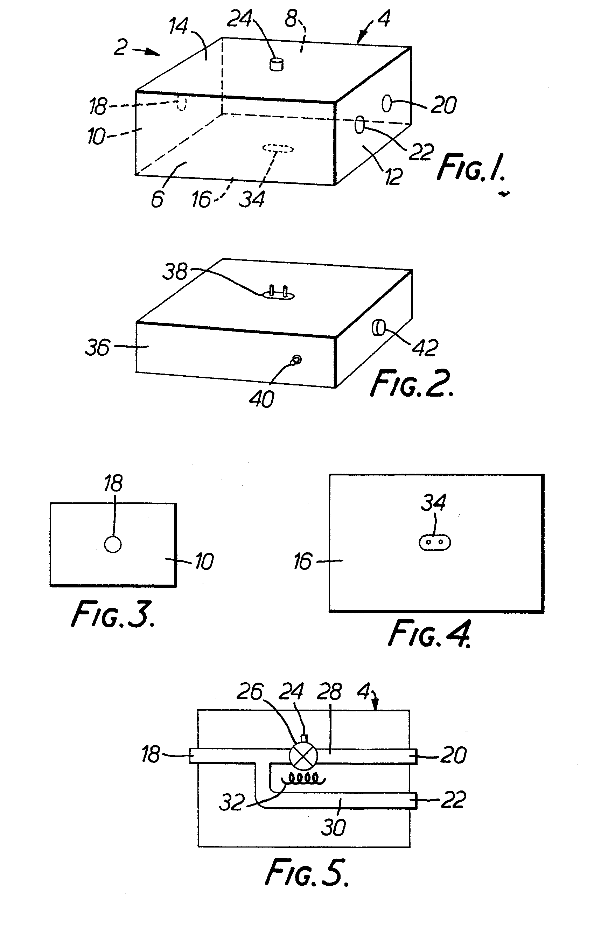

Referring to Figure 1, a resuscitation apparatus 2 includes a box-shaped housing 4. The longer opposed side walls of the box-shaped housing 4 are indicated by the reference numerals 6 and 8, and the shorter side walls by the reference numerals 10 and 12. (The side 10 is also shown in Figure 3). The top and bottom walls of the box or housing 4 are indicated by the reference numerals 14 and 16 respectively. (The bottom 16 is also shown in Figure 4). The housing 4 is conveniently formed of any suitable durable plastics material.

There is an inlet port 18 in the side wall 10 and first and second outlet ports 20 and 22 in the opposite side wall 12. The inlet port 18 is adapted to be connected to a source of respirable gas at high pressure (e.g. an oxygen cylinder fitted with a regulator). By the term 'high pressure' is meant a pressure higher than normal respirable pressures. The outlet ports 20 and 22 may comprise self-capping connectors of the Schraeder type or any other simple connecting device.

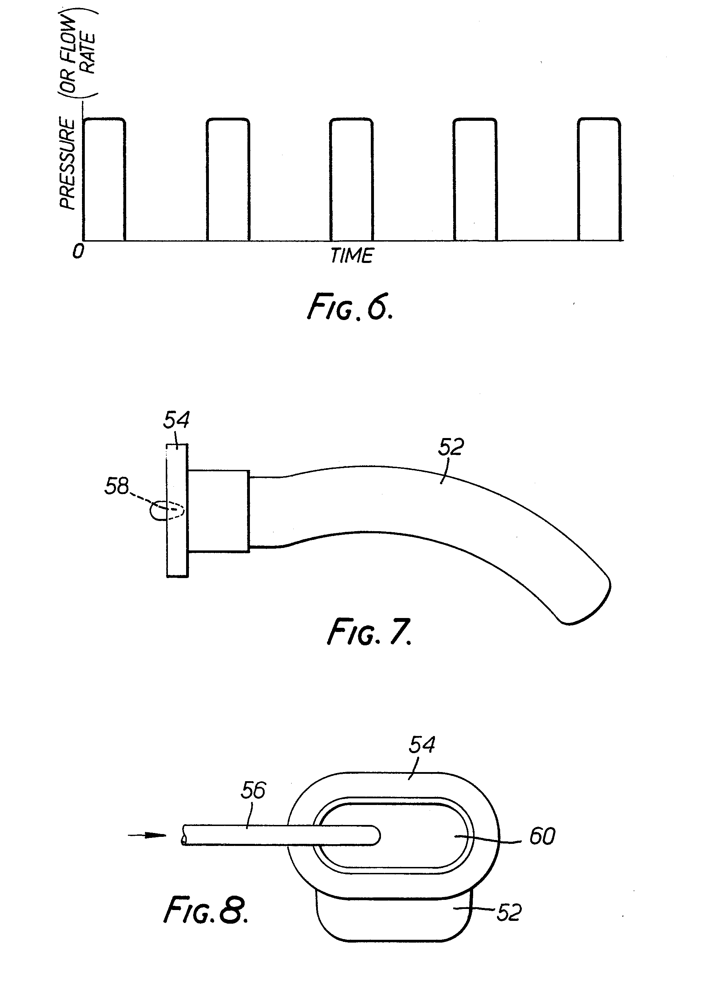

The housing 4 contains a solenoid (on-off) valve (not shown in Figure 1) which is alternatively operable by a push-button 24 projecting through the top wall 14. The valve is shown schematically in Figure 5 and is indicated by the reference 26. It is located in a main flow passage 28 connecting the inlet port 18 to the first outlet port 20. A tributary 30 from the main flow passage 28 extends from upstream of the valve 26 to the second outlet port 22.

The valve 26 is capable of being operated manually by the push-button 24 or automatically by the solenoid 32. If the valve is operating automatically the push-button 24 may be operated to override the solenoid 32. In order to keep down the total size of the resuscitation apparatus 2, it is desirable that the valve 26 be relatively small. The valve may, for example, be a Martonair (trade mark) valve having two valve positions and three ports (with one of the outlet ports blocked).

The solenoid 32 of the valve 26 is electrically connected to an electrical socket 34 in the bottom wall 16 of the housing 4. This socket is adapted to be mated with the complementary plug 38 of an electronic valve control box or device 36 (see Figure 2). The control box 36 typically has a switch 40 for switching on its own internal power supply (not shown) which may typically comprise rechargeable or disposable batteries. The control box 36 also has a control knob 42 for adjusting the frequency with which the valve 26 may be opened and closed. The control knob 42 is associated with electronic circuits capable of generating signals effective to open and close the valve 26 so as to supply to the outlet port 20 pulses of gas as shown in Figure 6.

As can be seen from Figure 6 the interval between the generation of each pulse and the next is twice as long as the duration of each pulse. Each pulse corresponds to the inspiratory phase of a patient's breathing and the interval between one pulse and the next to the expiratory phase of the patient's breathing. Accordingly, a constant inspiratory phase/expiratory phase ratio of 1:2 is given by operating the valve automatically using the control box. Moreover, by adjusting the control knob 42 the duration of each pulse may be adjusted to suit the particular patient without altering the inspiratory phase/expiratory phase ratio. Electronic control circuits capable of generating the necessary signals for such operation of the valve 26 are well known and will not be described in detail in this specification. Suffice it to state that typically, two integrated circuits are required, one for generating rapid electrical pulses, the other containing a gate means for selecting pulses. Typically, the control device may produce from 15 to 40 gas pulses per minute.

It is to be appreciated that instead of using the control box 36 the valve 26 may be operated manually by means of the push-button 28 to produce a pulsed gas supply such as that shown in Figure 6.

The first outlet port 20 may be connected to any suitable means for delivering the pulsed high pressure gas supply to the patient. The preferred means is an anatomically-shaped artificial airway as claimed and described in UK Patent Specification 1 558 171. Such an airway is shown in Figure 7 and 8. Connecting the airway shown in Figures 7 and 8 to the outlet port 20 of the apparatus shown in Figures 1 and 3 to 5 forms a complete manually-operable resuscitator. If the control box shown in Figure 2 is connected to the apparatus shown in Figures 1 and 3 to 5, the resuscitator may operate automatically.

The airway shown in Figures 7 and 8 consists basically of an open-ended tube 52 having at one end 60 an outwardly-directed flange 54. The tube 52 is intended to be fitted into a patient's mouth so that the tube overlies the patient's tongue and extends into the oro-pharynx. The flange 54 is intended to come into contact with either the patient's teeth or lips so as to prevent the patient from swallowing the airway and to limit the extent to which the airway projects into the patient's oro-pharynx.

Extending to the side of the flange 54 is a length of tubing 56 which is able to be connected to the outlet port 20 of the resuscitation apparatus 2. The end of the tubing which is remote from the outlet port 20 terminates in a nozzle (or primary injector) 58 which is shaped so as to inject a jet of gas at high-pressure into the interior of the tube 54. The nozzle 58 may be of one piece with the tubing 56. The tubing 56 and nozzle 58 may be of one piece with the flange 54 and therefore with the tube 52. Alternatively, the tubing 56 may be bonded or otherwise secured in a non-detachable manner to the flange 54.

When the patient is being resuscitated, the interior of the tube 52 is open to the atmosphere through the central opening 60 in the flange 54. The pulses of respirable gas issue as jets from the nozzle (or primary injector) 58 and entrain air from the atmosphere. Each jet of gas and the entrained air are typically mixed completely by the time the mixture reaches the distal end of the airway in the patient's oro-pharynx. As the flow of gas leaves the distal end of the injector it will impinge on the soft palate tending to drive it back on to the posterior pharyngeal wall narrowing the aperture between the oro-pharynx and naso-pharynx. With the soft palate in this position the oro-pharynx flares out like the throat of a venturi. Thus, the end of the airway in the throat constituted a secondary injector entraining additional air through the nose and around the airway in the mouth. There is only a relatively weak suction but the important result is that it is not necessary to pinch the nose or cover the mouth to ensure inflation. The existence of a low suction pressure in these zones can be demonstrated by the movement of a wisp of cotton wool.

Typically, at the distal end of the airway, with no back pressure, flows of 100 ml to 1000 ml per second can be obtained with different sized airways and nozzles 58. As the back pressure rises the flow rate decreases and flow will cease at a pressure of 30 to 40 cm $H_2O$

according to the dimensions of the airway and nozzle 58. Typically, with pulse of oxygen at 60 psi the oxygen concentration delivered to the patient may be between 40% and 60% by volume.

Although the combination of a resuscitation apparatus such as that illustrated in Figures 1 and 3 to 5 with the artificial airway shown in Figures 7 and 8 is a preferred arrangement according to the invention, the outlet port 20 of the resuscitation apparatus illustrated in Figures 1 and 3 to 5 may be connected to alternative devices for delivering pulses of respirable gas to the patient.

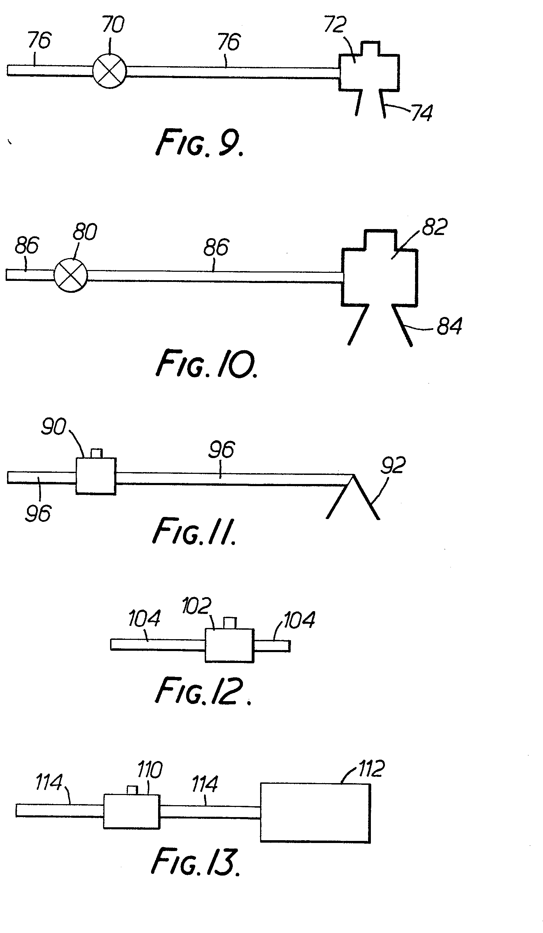

Examples of suitable alternative equipment are shown in Figures 9 and 10. The equipment shown in Figure 9 comprises a flow control valve 70, a non-return valve 72 suitable for an infant or child, and a face mask 74 suitable for an infant or child. In addition, there are suitable lengths of flexible connecting tubing 76. The equipment shown in Figure 10 is analogous to that shown in Figure 9 but is adapted for resuscitation of an adult. The equipment comprises a flow control valve 80, a non-return valve 82 suitable for an adult, and a face mask 84 suitable for an adult. In addition, there are suitable lengths of flexible connecting tubing 86.

Accordingly, by keeping with the resuscitation apparatus shown in Figures 1 and 3 to 5, a range of different sized equipment of the kind shown in Figures 7 and 8 and/or 9 and 10, all sizes of patient from neolates to large adults can be resuscitated.

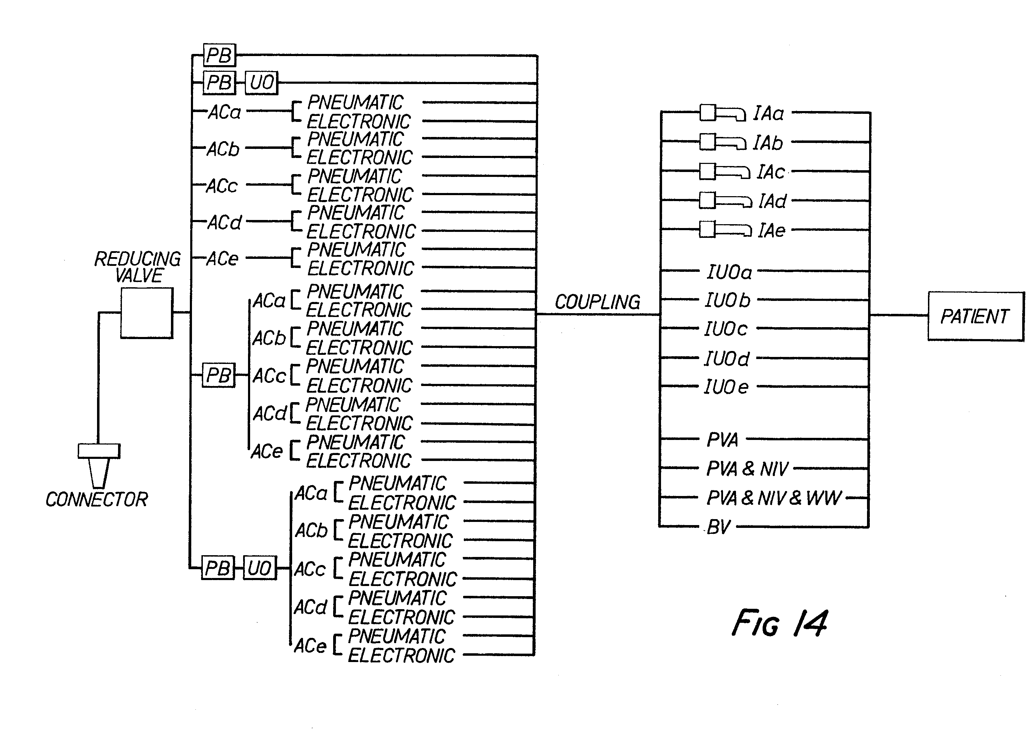

The continuous supply of respirable gas to the outlet port 22 of the resuscitation apparatus shown in Figure 1 may be emloyed in one of three alternative ways. First, a continuous flow of respirable gas may be supplied to assist the breathing of a patient who does not require resuscitation. Second, a pulsed flow of respirable gas may be created from the continuous flow and supplied to resuscitate a second patient. Third, a flow of gas may be supplied to an inflatable pillow so as to inflate it to a desired degree. The pillow may then be used to prop the head of a patient. Suitable equipment for these purposes is illustrated schematically in Figures 11, 12 and 13.

The equipment shown in Figure 11 comprises a flow control valve 90, an oxygen mask 92 and suitable lengths of flexible connecting tubing 96.

The equipment shown in Figure 12 comprises a manually (e.g. push-button) operable on-off valve 102 whose inlet is connected to a suitable length of flexible tubing 104. The outlet of the valve is connectible with the artificial airway shown in Figures 7 and 8 or with the equipment shown in Figure 9 or 10. The valve 102 may thus be operated manually to resuscitate one patient while another patient is resuscitated by means of pulses of respirable gas leaving the port 20 of the apparatus shown in Figure 1. Such pulses can be supplied automatically by means of the control box shown in Figure 2. Thus one suitably qualified person can use the apparatus shown in Figure 1 to resuscitate two patients simultaneously.

The apparatus shown in Figure 13 comprises a manually operable (e.g. push-button) valve 110, an inflatable pillow 112 of suitable material and suitable lengths of flexible connecting tubing 114. The valve 110 may be kept open until the pillow 112 has been inflated to a suitable degree and then closed.

The tubing employed in the airway shown in Figures 7 and 8 and the tubing employed in the equipment shown in Figures 9 to 13 may be provided at the end to be connected to the port 20 or 22 of the apparatus shown in Figure 1 with suitable connectors (not shown). Preferably, the ports 20 and 22 have connectors of different sizes from one another so that equipment intended to be connected to the port 20 cannot be connected to the port 22, and _vice versa_.

The equipment shown in Figures 11 to 13 may be kept or stored with the resuscitation apparatus shown in Figures 1 and 3 to 5, the control box shown in Figure 2 and suitable airways of the kind shown in Figures 7 and 8 (or equipment of the kind shown in Figures 9 and 10) to provide a resuscitation kit which may be used to resuscitate patients ranging from neonates to fully grown adults in a number of different situations. Moreover, hospitals, rescue services and ambulance services may select only those parts of the whole range of apparatus and equipment illustrated in the drawings so as to meet their own particular requirements.

Referring to Figure 14, the manufacturer of the resuscitation apparatus wouldhave in stock and purchasable individually the following modules:-

A standard connector to a gas supply and called "connector" in Figure 14.

A pressure reducing valve called "reducing valve"

A push-button manual control device for the reducing valve and reference "PB" and operable to give manually controlled pulses of respirable gas.

A manual control device referenced "UO" and operable to caused the reducing valve to give a continous, i.e. unpulsed, flow of respirable gas, usually oxygen.

A series of pneumatic automatic control devices for the reducing valve and referenced "ACa pneumatic" to "ACe pneumatic". ACa refers to a device giving a constant volume flow rate at a fixed respiration frequency and with a fixed inhalation/exhalation time ratio. ACb represents a device giving a constant flow rate with a variable frequency and a fixed inhalation/ exhalation time ratio. ACc refers to a device for giving a variable flow with a variable frequency and a fixed inhalation/exhalation time ratio. ACd refers to a device giving a constant flow with a variable frequency and a variable inhalation/exhalation time ratio. ACe represents a device for giving a variable flow with a variable frequency and a variable inhalation/exhalation time ratio.

A series of electronic automatic control devices referenced "ACa electronic" to "ACe electronic". The references ACa to ACe have the same meaning as in the case of the pneumatic devices.

A quick-connect and quick-release coupling referenced "coupling".

A series of injection airways referenced IAa to IAe and of various sizes.

A series of injection units designed for oxygen and referenced IUOa to IUOe and which can comprise face masks or endotracheal/tracheostomy tubes.

An adult patient valve referenced "PVA".

An adult patient valve combined with a non-inhalation valve and referenced "PVA/NIV".

An adult patient valve with a non-inhalation valve and a warning whistle and referenced "PVA/NIV/WW".

A baby patient valve referenced "BV".

The patient is also indicated in Figure 14.

A purchaser would buy the connector; the reducing valve; at least one of the valve control devices "PB", "UO" and "AC"; the coupling; and at least one of the respirable gas administering devices "IA", "IUO", "PVA", "PVA/NIV", "PVA/NIV/WW", and "BV". In practice, he would purchase a considerable part of the ranges available. At the place where the resuscitation apparatus is to be used, such as at a hospital, the connector, the reducing valve, a valve control device, the coupling, and an administering device would be assembled together, the valve control device and the administering device being selected according to the circumstances and the type of patient.

This method of assembling of the apparatus enables one apparatus with additional interchangeable modules to cope with a wide variety of patients in a wide variety of circumstances.

Particularly in the case of resuscitation apparatus applicable to new-born babies, it is especially advantageous for the apparatus to be so designed that the first few breaths can be at a high flow rate, in order to expand the lungs properly, but the following breaths at a low flow rate more corresponding to normal breathing. For this purpose, the manual control device PB is advantageously designed to open the reducing valve sufficiently to give a high rate of flow, whilst the automatic valve control devices AC are advantageously designed to give a low rate of flow from the valve.

Figures 15 to 17 show possible designs of the device ACb electronic or ACe electronic.

Referring to Figure 15, the device naturally requires a power source 100. This power source can be any supply of electrical energy, for example dry cells, rechargeable batteries or the mains. The power source 100 is connected to an oscillator 101, a ratio divider 102 and a solenoid-operated valve 103. The oscillator is a variable frequency oscillator and can be any circuit capable of producing a digital train of pulses, which may be varied in frequency by the setting of a control 104. The ratio divider 102 can be any circuit which may be programmed to operate the solenoid-operated valve 103 for 1 out of n of the incoming pulses from the oscillator 101. The process is cyclic and repeats every n pulses. The valve 103 comprises an elctro-mechanical actuator assembly which when energised allows a change in the state of flow of the gas to be controlled.

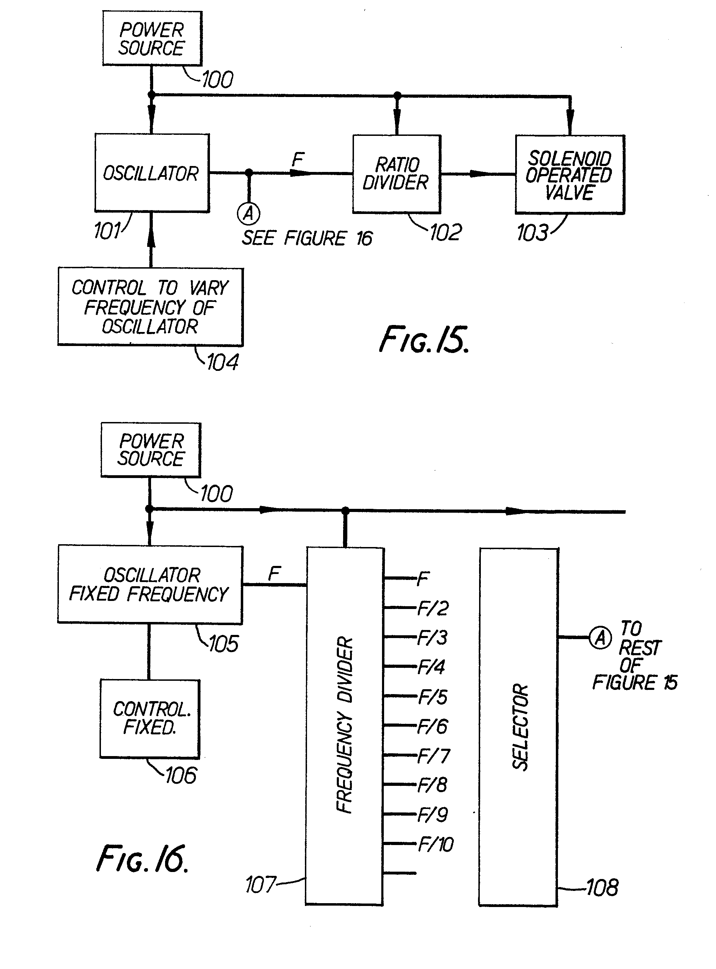

Referring to Figure 16, the oscillator, now referenced 105, is a fixed frequency oscillator with a fixed control 106. The output from the oscillator 105 is fed to a frequency divider 107 also connected to the power source 100. A selector 108 is used to select a desired frequency signal from the divider 107 and feed it to the ratio divider 102. The frequency divider 107 is a circuit which can take the incoming pulse train from the oscillator 105 and produce a series of frequency divided outputs.

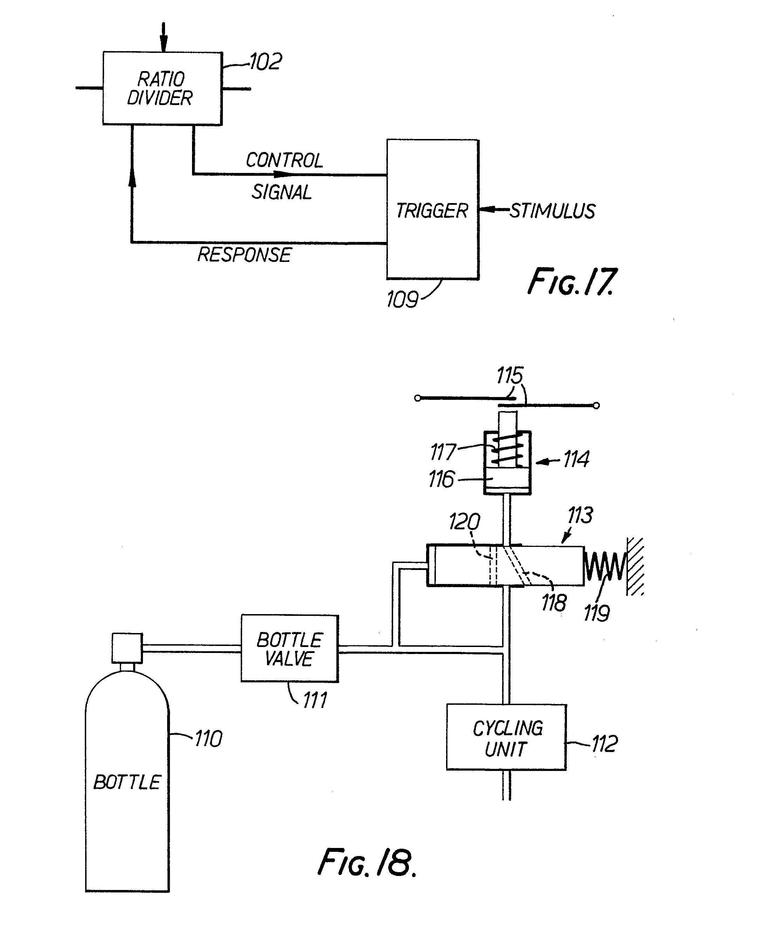

The circuit may be extended to allow for modification of the output wave form of the ratio divider 102 in response to a stimulus from an outside source. Such an arrangement is shown in Figure 17, wherein a control signal from the ratio divider 102 is fed to a trigger device 109. On receipt of an external stimulus, the trigger device 109 emits an electrical response signal to modify the output from the ratio divider 102.

In some circumstances, it is particularly advantageous if the power supply to the electronic control device is not energised until the gas supply is switched on and is switched off immediately the gas supply is switched off. An arrangement for achieving this is illustrated in Figure 18. A gas bottle 110 is shown connected via a bottle valve 111 to a cycling unit 112. Also arranged downstream of the valve 111 is a slide valve 113 and, downstream thereof, a piston-in-cylinder device 114 arranged to control a pair of electrical contacts 115 arranged in the power supply circuit of the electronic control device. In the condition shown, the piston 116 of the device 114 has been pressed by a return spring 117 into a position corresponding to an open position of the contacts 115, the gas in the cylinder having been expelled via an exhaust port 118 of the valve 113. On switching on of the bottle valve 111, the downstream gas pressure of the valve 111 increases and is applied to the left-hand end of the slide valve 113 to press it against the action of a return spring 119 into a position in which a through passage 120 of the valve 113 communicates the gas pressure downstream of the valve 111 to the device 114, so forcing the piston 116 against the action of the spring 117 to close the contacts 115. On closing of the bottle valve 111, the pressure downstream of the valve 111 falls and the spring 119 returns the valve 113 into its exhaust condition shown.

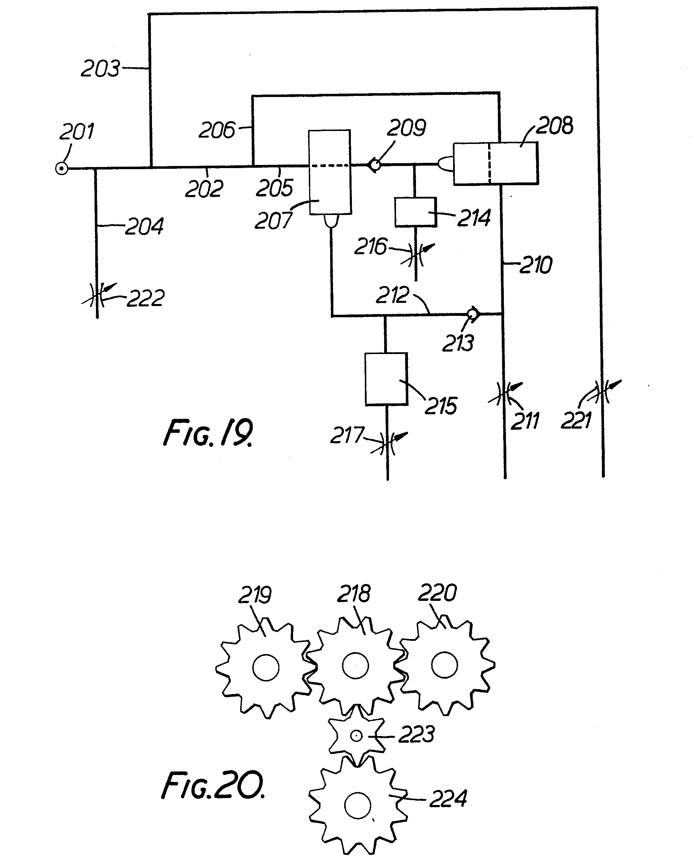

Referring to Figure 19, the pneumatic circuit part shown therein includes three conduits 202, 203 and 204 which are supplied from the nutrient gas supply 201. The conduit 202 divides into two conduits 205 and 206, of which the conduit 205 leads to the inlet of a normally-open, pilot-controlled valve 207, and the conduit 206 to a normally-closed, pilot-controlled valve 208. On commencement of flow from the supply 201, the nutrient gas passes straight through the valve 207 and through a non-return valve 209 to a pilot pressure chamber of the valve 208, so enabling nutrient gas to flow through the valve 208 from the conduit 206 to a conduit 210 which leads _via_ an adjustable flow restrictor 211 to a patient valve, which is shown in Figure 21. Nutrient gas in the conduit 210 can flow _via_ a conduit 212 containing a non-return valve 213 to a pilot pressure chamber of the valve 207. Nutrient gas is trapped in the pilot conduits by the non-return valves 209 and 213 and fills reservoirs 214 and 215 connected upstream of the non-return valves 209 and 213, respectively. The reservoirs 214 and 215 can empty only through respective adjustable bleeds 216 and 217. The adjustable bleeds 216 and 217 and the adjustable flow restrictor 211 are linked by some non-pneumatic means such that their throughflow cross-sections remain in a fixed ratio relative to one another. An example of such non-pneumatic means is shown diagrammatically in Figure 20, in which inter-meshing gear wheels 218, 219 and 220 are connected to adjusting shafts of the restrictor 211 and the bleeds 216 and 217, respectively. Inserted in the conduit 203 is a manually adjustable flow restrictor 221. The conduits 203 and 210 downstream of the

0097060

-20-

restrictors 221 and 211, respectively, may remain independent of each other, or may be joined together to give higher flow rates. The conduit 204, which contains a manually adjustable flow restrictor 222, can give a continuous flow of nutrient gas to the patient. The outlets from the flow restrictors 211, 221 and 222 are preferably in the form of quick-release couplings to allow easy change from one mode of treatment to another. These three outlets may all be used simultaneously on three respective patients, or two of these outlets may be used on two respective patients, or one outlet on a single patient.

In a case where the conduits 203 and 210 are joined together downstream of the restrictors 221 and 211, respectively, the restrictor 221 may be arranged to be non-pneumatically linked for adjustment with the restrictor 211, such linkage being instead of or as a selectable alternative to manual adjustment of the restrictor 221. As shown in Figure 20, such linkage may be achieved by means of connecting to the gear wheel 211 by way of an intermediate gear wheel 223, a gear wheel 224 connected to an adjusting shaft of the restrictor 221.

The apparatus described with reference to Figures 19 and 20 has an advantage of being a single apparatus usable for oxygen therapy (or therapy utilizing another gaseous substance), manual intermittent positive pressure ventilation, or automatic intermittent positive pressure ventilation, and enabling up to three patients to be treated at the same time. For example, in midwifery, the baby may need manual or automatic intermittent positive pressure ventilation, while the mother has oxygen therapy.

Although the pneumatic circuit shown in Figure 19 comprises two variable bleeds 216 and 217 and two fixed-volume reservoirs 214 and 215, it could instead comprise two fixed-flow-rate bleeds and two

-21-

variable-volume reservoirs. Alternatively, it could have two variable bleeds giving coarse control of the flow rates and two variable reservoirs giving fine control of the flow rates.

It will be appreciated that the reservoir 214 and the bleed 216 determine the inhalation time period, whilst the reservoir 215 and the bleed 217 determine the exhalation time period.

In most normal circumstances, the ratio of the inhalation time period to the exhalation time period is advantageously about 1:2. Owing to the non-pneumatic linkage between the restrictor 211 and the bleeds 216 and 217, not only is it possible to ensure that this ratio remains constant, but also the respiration frequency varies automatically with adjustment of the flow rate to the patient, so that the apparatus is usable with simple adjustment for either a large adult or a small child. In practice, with a decrease in the flow rate to the patient, there is an increase in the ventilation frequency, and vice-versa.

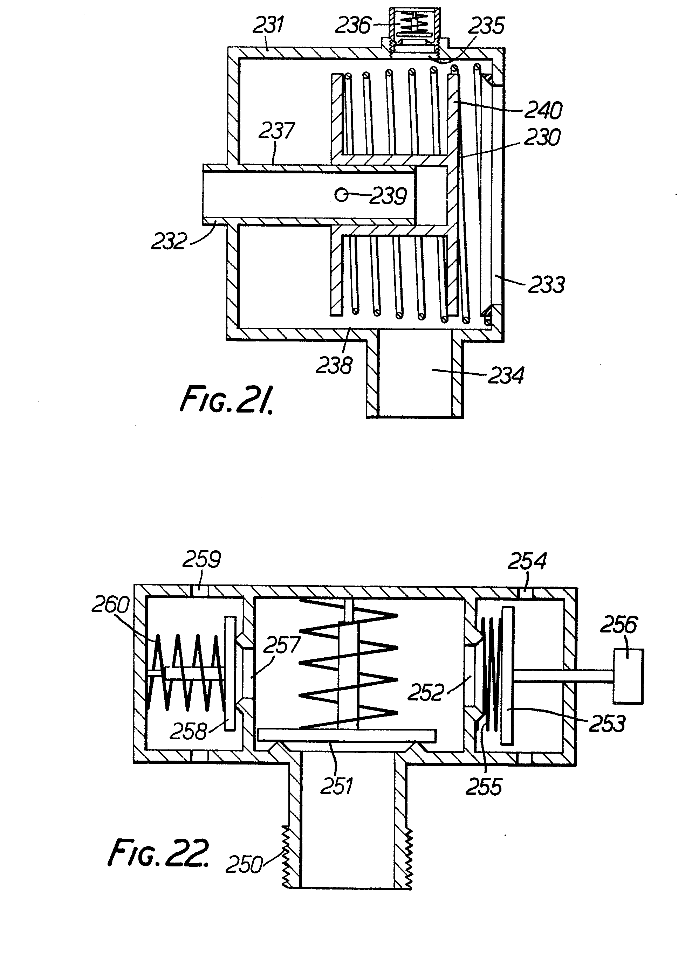

The patient valve shown in Figure 21 comprises a bobbin 230 which slides with clearance in a shell 231 which has an inlet 232 for nutrient gas and an outlet 233 for exhaled gas. The shell 231 also comprises an opening 234 leading to the patient and an opening 235 receiving a screw-in positive pressure relief valve 236. The nutrient gas entering the inlet 232 flows along an inlet tube 237 encircled by the bobbin 230. The pressure of the gas urges the bobbin 230 against the action of a return spring 238 into a position in which it seals the outlet 233. The pressure of the nutrient gas in the bobbin 230 is the inlet pressure of that gas, until a hole 239 through the tube 237 is exposed by the movement of the bobbin 230 towards its position sealing the outlet 233, whereupon the pressure in the bobbin 230 drops, but at the same time the pressure against the inside face of the closure plate 240 of the bobbin 230 rises, and this constitutes a substantial part of the sealing force, especially at low flow rates. Exposure of the hole 239 allows high flow rates of nutrient gas to be transmitted to the patient. The two flanges of the bobbin are of equal external diameter to each other. The valve 236 is a screw-in unit which can be exchanged for another simple valve or a compound valve device such as that illustrated in Figure 22, which is for neonates.

Referring to Figure 22, the valve device includes a threaded inlet stem 250 which is screwed into the opening 235. The device also includes a primary valve 251 which opens at comparatively low pressure in the stem 250, for example 30 cm. $H_2O$. The gas from the valve 251 exhausts through a port 252 of another valve 253 and thence through openings 254 to atmosphere. The valve 253 is normally kept open by its spring 255. However, when a knob 256 of the valve 253 is depressed, the port 252 is sealed and the gas is then voided through a port 257 of a valve 258 and thence through openings 259 to atmosphere. The closure member of the valve 258 is urged to close the port 257 by its spring 260, which is set to give an opening pressure for the valve 258 corresponding to a comparatively high pressure in the stem 250, for example 55 cm. $H_2O$. An advantage of this valve device is that it is possible to obtain positive pressure relief at two differing positive pressures without disturbing the setting of the valve closure springs.

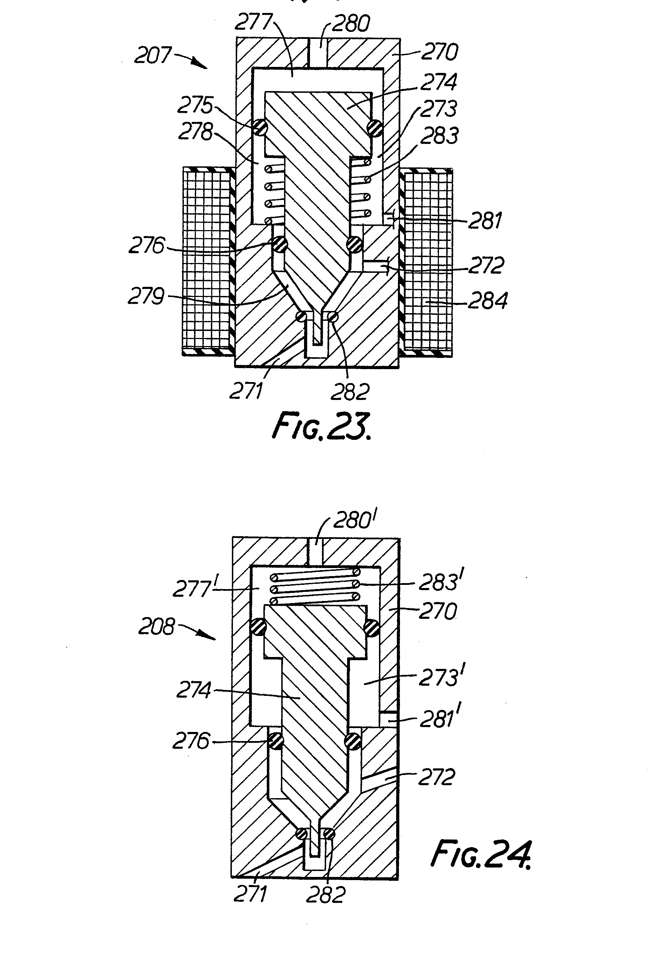

The normally-open, pilot-controlled valve 207 is seen in more detail in Figure 23. It comprises a valve housing 270 with a nutrient gas inlet 271 and a nutrient gas outlet 272 connected in the conduit 205. The housing 270 has a hollow interior 273 to and from which lead the inlet 271 and the outlet 272, the interior 273 containing co-axially a valve closure member 274 in the shape of a pin with a cylindrical head and a conical tip. A seal 275 around the head of the member 274 and an annular seal 276 encircling the shank of the member 274 divide the interior 273 into a pilot pressure chamber 277, an atmospheric pressure chamber 278, and a nutrient gas line pressure chamber 279. The chamber 277 communicates _via_ a connection 280 with the conduit 212, the chamber 278 with atmosphere through an aperture 281, and the chamber 279 with the inlet 271 and the outlet 272. Near the end of the chamber 279 further from the chamber 277 the housing 270 carries co-axially an annular seal 282 which is of considerably smaller diameter than is the seal 276. An annular compression spring 283 encircling the shank of the member 274 and acting between the housing 270 and the head of the member 274 urges the member 274 into a fully open position. In the open condition of the valve 207, the line pressure in the chamber 279 is applied over the relatively small effective area of the member 274 at its tip, whilst the pilot pressure in the chamber 277 is applied over the relatively large effective surface area of the member 274 at its head. Thus the pilot pressure may be well below the line pressure. The valve member 274 is kept open by the spring 283 which is relatively weak. As the member 274 nears the seating seal 282, the conical shape of its tip gives a decreasing surface area exposed to the full line pressure, so giving a rapid change of state from the open condition to the closed condition. Moreover, in the closed condition, the line pressure is applied over only the relatively very small effective area defined by the seal 282. As the member 274 begins to open, the shape of the tip gives an increasing area exposed to the full line pressure, so giving a rapid change of stage from the closed condition to the open condition.

The pilot-controlled normally-closed vlave 208 shown in Figure 24 is identical to the valve 207, except that the aperture 280' leads to atmosphere, so that the chamber 277' is the atmospheric chamber; the aperture 281' is connected to the conduit 212, so that the chamber 273' becomes the pilot pressure chamber; and the spring 283' is situated in the chamber 277' and so urges the member 274 into its fully closed condition. In the closed condition of the valve 208, the seal 282 defines the relatively very small surface area over which the line pressure acts on the member 274. As the member 274 begins to open, the relatively large area now exposed to the line pressure and defined by the seal 276 compensates for any drop in the line pressure. The pilot pressure in the chamber 273' still acts over a yet larger area, so that the valve responds to a relatively low pilot pressure.

If it is desired to permit electrical operation of the valve 207 or 208, an inductive coil 284 can encircle the housing 270, which would be made of non-ferrous material, whilst the member 274 would be made of ferrous material. Such valve device may be used with either pneumatic or electrical pulsing, whereby it is possible to have an electrical override of the pneumatic circuit, or pneumatic override of the electrical circuit.

By providing a longer shank of the member 274, the member can be used to open and close further control inlets and outlets, thereby providing a three-, four- or more- port valve according to need, but keeping the principle of an inlet pilot.

The bobbin 230 has its two flanges of virtually equal external diameter, so that the ratio of the exposed areas of the flanges is not a significant factor in the motion of the bobbin.

CLAIMS

1. Resuscitation apparatus comprising an inlet (18, 203) connectible to a source (201) of respirable gas, an outlet (20) connectible to a patient's respiratory system, duct means (28) interconnecting said inlet (18, 203) and said outlet (20), and a valve (26, 221) interposed in said duct means (28) and operable manually by manually operable means (24) connected to said valve (26, 221) characterized in that said valve (26, 221) is also operable selectively automatically by automatically operable means (32, 223, 224) connected to said valve (26, 221).

2. Apparatus according to claim 1, wherein said automatically operable means (32,223, 224) comprises an electronic control device (36) and said valve (32, 223, 224) is operable automatically electrically.

3. Apparatus according to claim 1 or 2, wherein said automatically operable means (32, 223, 224) operates said valve (26, 221) to give a constant inspiratory phase/expiratory phase ratio.

4. Apparatus according to any preceding claim, and further comprising adjusting means (42, 104, 107, 108) arranged to adjust the duration of the inspiratory phase produced by said automatically operable means (32, 223, 224)..

5. Apparatus according to any preceding claim, wherein said manually operable means (24), on operation thereof, overrides said automatically operable means (32, 223, 224)..

6. Apparatus according to any preceding claim, and further comprising a second outlet (22, 204) communicating with said duct means (28) at a location upstream of said valve (26, 221).

7. Apparatus according to claim 6, and further comprising a manually operable valve (222) connected downstream of said second outlet (22, 204).

8. Apparatus according to any preceding claim, and further comprising a housing (4) containing said valve (26, 221) and said duct means (28), said automatically operable means (32, 223, 224) including a connector (34) in a wall (16) of said housing (4) for connecting the valve (26, 221) to an external automatic control device (36).

9. Apparatus according to any preceding claim, and further comprising a modular gas administering device (52, 74) connected downstream of said outlet (20) for administering said gas to said system, said manually operable means (24) comprising a modular manual control device, and said automatically operable means (32, 223, 224) comprising a modular automatic control device (any one of Figures 15 to 19).

10. A method of assembling a resuscitation apparatus, characterized in that it comprises selecting a control device from a range of modular control devices (PB, 36, Figures 15 to 19), connecting said control device to a respirable gas flow control valve (26, 221) to be operated by said control device, selecting a respirable gas administering device from a range of modular respirable gas administering devices (52, 74 etc.), and connecting said administering device to downstream of said valve (26, 221) for administering said gas to a patient's respiration system.

11. A method according to claim 10, and further comprising selecting an automatic control device from said range of modular control devices (PB, 36, Figures 15 to 19) and connecting said automatic control device to said valve (26, 221), the first-mentioned control device being a manual control device (PB).

12. A collection of parts from which a resuscitation apparatus can be assembled, characterized in that it comprises a collection of modular devices from a selection from which said resuscitation apparatus can be assembled, comprising a respirable gas flow valve (26, 221), a range of modular control devices (PB, 36, Figures 15 to 19) each connectible to said valve (26, 221) for operating the same, and a range of modular respirable gas administering devices (52, 74 etc.) connectible to downstream of said valve (26, 221) for administering said gas to a patient's respiratory system.

13. A collection according to claim 12, wherein said range of modular control devices (PB, 36, Figures 15 to 19) comprises a manual control device (PB), a pneumatic automatic control device (Figure 19) and an electrical automatic control device (Figure 15).

14. A collection according to claim 12 or 13, wherein said range of modular administering devices (52, 74 etc.) comprises an injection airway (IA), an injection unit (IUO) designed for oxygen, an adult patient valve (PVA), and a baby patient valve (BV).

15. A resuscitation apparatus, comprising a respirable gas flow valve (26, 221), a control device (PB, 36, Figures 15 to 19) connected to said valve (26, 221) for operating the same, and a respirable gas administering device (52, 74 etc.) connected to downstream of said valve (26, 221) for administering said gas to a patient's respiratory system, characterized in that said control device (PB, 36, Figures 15 to 19) and said administering device (52, 74, etc.) are modular.

16. An apparatus according to claim 15, and further comprising a modular automatic control device (36, Figures 15 to 19) connected to said valve (26, 221) for operating the same, the first-mentioned control device being a modular manual control device (PB).

17. Resuscitation apparatus comprising an inlet (18, 203) connectible to a source of respirable gas, an outlet (20) connectible to a patient's respiratory system, duct means (28) interconnecting said inlet (18, 203) and said outlet (20), a valve (26, 221) interposed in said duct means (28) and operable automatically, and electrical means (36) connected to said valve (26,221) and arranged to operate said valve (26,221) automatically, characterized in that said electrical means (36) includes electrical switching means (114, 115) arranged to detect the presence or absence of said gas under pressure upstream of said valve (26, 221) and accordingly to energize or de-energize said electrical means (36).

18.    A respiratory apparatus including first and second variable flow control valves (211, 221) which serve to control respective flows of gaseous fluid therethrough, characterized in that a non-pneumatic adjusting linkage (218, 223, 224) interconnects the valves (211, 221) so that adjustment of the first valve (211) is accompanied by adjustment of the second valve (221).

19.    A valve device, comprising an inlet (271) for fluid, an outlet (272) for said fluid, valve closure means (274) arranged to control flow of said fluid from said inlet (271) to said outlet (272), and a pilot pressure chamber (277, 273) bounded by said valve closure means (274), characterized in that the effective area of said valve closure means (274) exposed to the pressure of said fluid between said inlet (271) and said outlet (272) is less than the effective area of said valve closure means (274) exposed to the pressure in said chamber (277, 273').

20.    A valve device comprising a valve housing (231), a valve closure member (230) movable in said housing (231) from an open position to a closed position, an inlet (232) to said housing (231) for fluid, and an outlet (234) from said housing (231) for fluid, characterized in that, as said member (230) moves from said open position to said closed position, initially fluid can flow via a first path from said inlet (232) to a limited degree and via a second path (239) from said inlet to no more than a limited extent, but subsequently said member (230) enables flow of said fluid _via_ said second path (239) from said inlet 232) to greater than said limited extent, whereby said fluid can flow from said inlet (232) to an increased extent.

21.    A valve device comprising a valve housing (231), a valve closure member (230) movable in said housing (231) from an open position to a closed position, a tubular inlet (237) to said housing (231) for fluid, and an outlet (234) from said housing (231) for fluid, said member (230) being in the form of a bobbin (230) closely encircling said tubular inlet (237), characterized in that said bobbin (230) includes first and second flanges of substantially equal external dimension transverse to the bobbin axis.

22.    A valve device comprising a first valve including a first valve closure member (251) arranged to open automatically upon occurrence of a predetermined pressure differential thereacross, and a second valve which includes a second valve closure member (253) and through which the first valve exhausts when the second valve closure member (253) is open, characterized in that the second valve closure member is selectively closable and in that there is a third valve which includes a third valve closure member (258) arranged to open automatically upon occurrence of a predetermined pressure differential thereacross and through which the first valve exhausts when the second valve closure member (253) is closed, the arrangement being such that that pressure upstream of said first valve at which said third valve opens is higher than that pressure upstream of said first valve at which said first valve opens.

FIG.1.

FIG.2.

FIG.3.

FIG.4

FIG.5.

PRESSURE (OR FLOW RATE)

TIME

*FIG. 6.*

54

52

58

*FIG. 7.*

54

56

60

52

*FIG. 8.*

FIG. 9.

FIG. 10.

FIG. 11.

FIG. 12.

FIG. 13.

CONNECTOR

REDUCING VALVE

PB
PB — UO

ACa — PNEUMATIC / ELECTRONIC
ACb — PNEUMATIC / ELECTRONIC
ACc — PNEUMATIC / ELECTRONIC
ACd — PNEUMATIC / ELECTRONIC
ACe — PNEUMATIC / ELECTRONIC

PB
ACa — PNEUMATIC / ELECTRONIC
ACb — PNEUMATIC / ELECTRONIC
ACc — PNEUMATIC / ELECTRONIC
ACd — PNEUMATIC / ELECTRONIC
ACe — PNEUMATIC / ELECTRONIC

PB — UO
ACa — PNEUMATIC / ELECTRONIC
ACb — PNEUMATIC / ELECTRONIC
ACc — PNEUMATIC / ELECTRONIC
ACd — PNEUMATIC / ELECTRONIC
ACe — PNEUMATIC / ELECTRONIC

COUPLING

IAa
IAb
IAc
IAd
IAe

IUOa
IUOb
IUOc
IUOd
IUOe

PVA
PVA & NIV
PVA & NIV & WW
BV

PATIENT

FIG 14

4/9

0097060

0097060

**POWER SOURCE** ⎯ 100

**OSCILLATOR** 101

F Ⓐ SEE FIGURE 16

**RATIO DIVIDER** 102

**SOLENOID OPERATED VALVE** 103

**CONTROL TO VARY FREQUENCY OF OSCILLATOR** 104

FIG. 15.

**POWER SOURCE** ⎯ 100

**OSCILLATOR FIXED FREQUENCY** 105

**CONTROL. FIXED.** 106

F

**FREQUENCY DIVIDER** 107

F
F/2
F/3
F/4
F/5
F/6
F/7
F/8
F/9
F/10

**SELECTOR** 108

Ⓐ TO REST OF FIGURE 15

FIG. 16.

RATIO DIVIDER 102

CONTROL SIGNAL

TRIGGER ← STIMULUS

RESPONSE

109

Fig.17.

115

117 116 114

120 113

118 119

BOTTLE VALVE 111

CYCLING UNIT 112

110

BOTTLE

Fig.18.

FIG. 19.

FIG. 20.

231   236   235

237

240

230

239

232

233

238   234

**Fig.21.**

259   254

260

256

257

252

253

258

255

251

**Fig.22.** 250

277 280 270 207 275 274 273 278 283 276 281 279 272 284 271 282

**FIG.23.**

280' 277' 283' 270 208 273' 274 281' 276 272 271 282 **FIG.24.**